# EUROPEAN PATENT APPLICATION

(11) **EP 4 803 111 A1**
(43) Date of publication of application: **09.09.2026**
(21) Application number: 24886060.3
(22) Date of filing: 11.10.2024
(51) Int. Cl.: A61L 27/56, A61L 27/18, A61F 2/30, B33Y 80/00

(54) **POROUS THREE-DIMENSIONAL MENISCUS SCAFFOLD AND BIODEGRADABLE MEDICAL DEVICE COMPRISING SAME**

(30) Priority: 03.11.2023 KR 20230151068
(71) Applicant: Samsung Life Public Welfare Foundation, Seoul 04348 (KR)
(72) Inventor: WANG, Joon Ho, Seoul 06351 (KR); SEO, Jiyun, Seoul 06351 (KR)
(74) Representative: Plougmann Vingtoft a/s
(86) International application number: PCT/KR2024/015428
(87) International publication number: WO 2025/095385

(57) **Abstract**

The present invention relates to a porous three-dimensional meniscus scaffold and a biodegradable medical device comprising the same, and according to the present invention, an external force applied to the meniscus scaffold can be more stably and uniformly distributed by appropriately controlling a ratio of inter-fiber spacings within the meniscus scaffold, and as a result, a biodegradable medical device comprising the meniscus scaffold can exhibit improved structural stability.

## Description

### TECHNICAL FIELD

This application claims priority to Korean Patent Application No. 10-2023-0151068 filed with the Korean Intellectual Property Office on November 3, 2023, and the disclosures of the patent application are incorporated herein by reference.

The present invention relates to a porous three-dimensional meniscus scaffold and a biodegradable medical device comprising the same, and more particularly, to a porous three-dimensional meniscus scaffold having a more stable structure by appropriately controlling a ratio of inter-fiber spacings within the meniscus scaffold and a biodegradable medical device comprising the same.

### BACKGROUND ART

The meniscus serves as a cushion within the knee joint, and is two C-shaped fibrocartilage complexes found between condyles of a femur and a tibial plateau of a tibia. The meniscus plays important roles in load transfer, load distribution, shock absorption, joint stability, and lubrication of the knee.

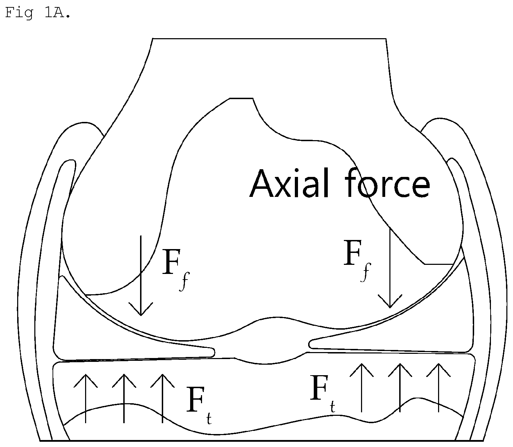

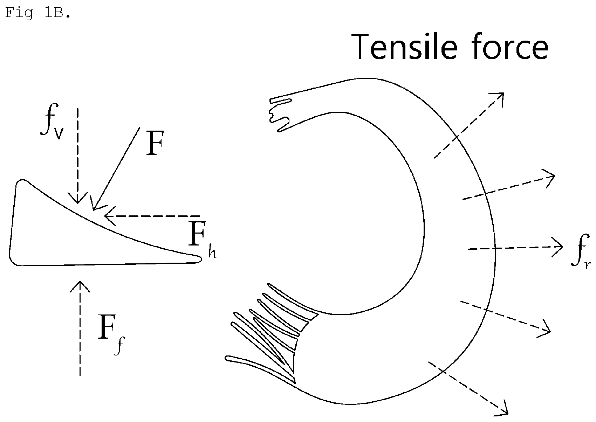

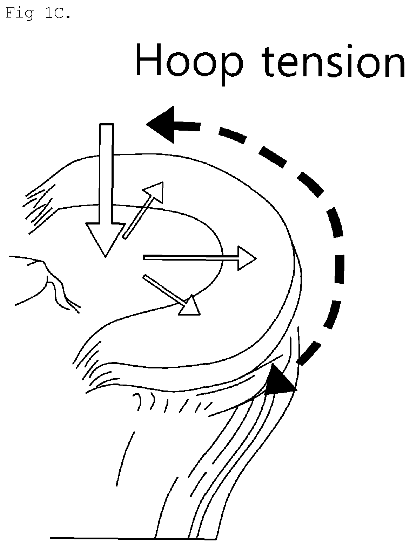

FIGS. 1A to 1C are drawings showing an axial force, a tensile force, and a hoop tension applied to a meniscus. Referring to FIGS. 1A to 1C, the meniscus has to evenly distribute a force while converting a compressive force applied as an axial force from a femur into a tensile force. At this time, a force pushed outward in a radial direction acts on the meniscus, and at this time, a hoop tension resisting this force has to be well maintained. In order for the hoop tension to be well maintained, strong fibers have to be arranged in a circumferential direction like a normal meniscus. A compressive force in an axial direction applied from a femur pushes the meniscus outward while being converted into a tensile force, and it is very important that the meniscus is not pushed outward.

Such damage of the meniscus is caused by degenerative changes of cartilage itself, various exercise activities, trauma, and the like, and is known as having the highest frequency among knee joint injuries. The meniscus is a structure within the human body, but one-third of a central portion has no blood vessels (white zone), only about one-third of a peripheral portion has blood vessels (red zone), and it is unclear whether blood vessels are present in an intermediate one-third thereof (red-white zone). When the meniscus is damaged, in the red zone which is a peripheral portion in which blood supply is relatively advantageous, healing is possible, and thus a repair may be performed, and in a case of a white zone of a central portion in which healing is not achieved because a blood vessel is not present, a meniscectomy may be performed. Even if there is rupture of the meniscus, repairing if possible is the best treatment, but when the rupture is rupture of a portion having no blood vessel or when degenerative changes have already significantly progressed so that repairing is impossible, a meniscectomy has to be performed. After performing a meniscectomy, when a defect of the meniscus is severe, an allogeneic meniscus transplantation may be performed for restoration of a structure. In a case of the allogeneic meniscus transplantation, although rare, there is a possibility of infection, accurate size selection of a transplant may be difficult, and because it is a human body tissue, the supply is often limited.

Furthermore, recently, research on implanting a natural or synthetic polymer scaffold in the form of a meniscus is being conducted. When an artificial tissue made by using a biomaterial scaffold used in tissue engineering is implanted into a body, in an initial stage of implantation into the body, implanted tissue cells do not die within a body due to the scaffold and provide a basis for maintaining original functions. And, as time elapses, a biodegradable polymer gradually disappears, and it has to be possible to form a tissue having the same form and function as a natural tissue composed of only implanted cells sufficiently adapted inside the body.

Meanwhile, in order to apply as a medical device, it is important to evenly distribute a force while converting an axial force acting on a meniscus scaffold into a tensile force, and for this, research and development on a stable structure of elements constituting the meniscus scaffold is necessary.

### DISCLOSURE OF INVENTION

### Technical Problem

A technical problem to be achieved by the present invention is to provide a porous three-dimensional meniscus scaffold capable of stably and uniformly distributing a load or force applied to the meniscal scaffold.

In addition, a technical problem to be achieved by the present invention is to provide a biodegradable medical device comprising the porous three-dimensional meniscus scaffold described above.

The technical problem to be solved by the present invention is not limited to the above-described problem, and another problem not mentioned herein will be clearly understood by a person skilled in the art from the following description.

### Solution to Problem

According to embodiments of the present invention for achieving the above-described problem, there is provided a porous three-dimensional meniscus scaffold comprising: a plurality of first axial direction layers including one or more first axial direction fibers spaced apart from each other at predetermined intervals along a first axial direction; and a plurality of second axial direction layers including one or more second axial direction fibers spaced apart from each other at predetermined intervals along a second axial direction, wherein the first axial direction layers and the second axial direction layers are stacked while crossing each other, wherein, in an (N+1)th first axial direction layer (where N is a natural number of 1 or more, the same applies hereinafter), an arbitrary first axial direction fiber (a) is present, and in an Nth first axial direction layer, a first axial direction fiber (b) first adjacent to the arbitrary first axial direction fiber (a) and a first axial direction fiber (c) second adjacent to the arbitrary first axial direction fiber (a) are present, assuming that the first axial direction fiber (a) is vertically moved to the Nth first axial direction layer as (a'), a ratio of a spacing between the (a') and the (b) to a spacing between the (a') and the (c) is in a range of 1:9 to 3:7.

At this time, the arbitrary first axial direction fiber and the second axial direction fiber may be stacked orthogonally to each other.

And, the (b), the (a'), and the (c) may be positioned in order from a peripheral side of the porous three-dimensional meniscus scaffold toward a central side.

In addition, the first axial direction fibers and the second axial direction fibers may independently comprise at least one polymer selected from the group consisting of polycaprolactone (PCL), Polyglycolic acid (PGA), Polylactic acid (PLA), Poly(lactic-co-glycolic) acid (PLGA), polyurethane (PU), and polydioxanone.

And, a width of pores in the porous three-dimensional meniscus scaffold may be 1 mm to 2 mm, and a height of the pores may be 0.1 mm to 0.5 mm.

In addition, a porosity of the porous three-dimensional meniscus scaffold may be 70% to 85%.

And, the first axial direction fiber and the second axial direction fiber may independently have a width of 270 µm to 330 µm.

In addition, the porous three-dimensional meniscus scaffold may further comprise a circumferential fiber formed to surround an outer surface thereof.

And, the porous three-dimensional meniscus scaffold may be manufactured through a 3D printer.

Meanwhile, according to another embodiment of the present invention, there is provided a biodegradable medical device comprising the above-described porous three-dimensional meniscus scaffold according to the present invention.

### Advantageous Effects of Invention

According to one embodiment of the present invention, an external force applied to a meniscus scaffold can be more stably and uniformly distributed by appropriately controlling a ratio of inter-fiber spacings within the meniscus scaffold.

As a result, a biodegradable medical device comprising the meniscus scaffold can exhibit improved structural stability.

Meanwhile, the effects of the present invention are not limited to those described above and include all effects that can be inferred from the detailed description of the invention and the features set forth in the claims.

### BRIEF DESCRIPTION OF DRAWINGS

FIG. 1A is a drawing showing an axial force applied to a meniscus, FIG. 1B is a drawing showing a tensile force acting on the meniscus, and FIG. 1C is a drawing showing a hoop tension acting on the meniscus.
FIG. 2A is a drawing showing a C-shaped porous three-dimensional meniscus scaffold according to one embodiment of the present invention, which mimics a human meniscus, and FIG. 2B is a drawing showing dimensions of the meniscus scaffold shown in FIG. 2A.
FIG. 3 is a drawing showing a cross-sectional view of a meniscus scaffold according to one embodiment of the present invention, and schematically showing an arrangement relationship among an arbitrary first axial direction fiber (a) of an (N+1)th first axial direction layer and a first axial direction fiber (b) and a first axial direction fiber (c) of an Nth first axial direction layer.
FIG. 4 is a drawing schematically showing a magnitude of forces resisting in outward and inward direction when an external force acts on the meniscus scaffold of FIG. 3.
FIG. 5 is a drawing showing a circular center line of an STL 3D model of a meniscus scaffold according to one embodiment of the present invention through image processing, and showing a pore structure formed inside.
FIG. 6 is a drawing showing that an alternate ratio for having structures of various pores in order to evaluate stress concentration and stability according to a structure of pores formed inside a meniscus scaffold is defined, and showing a result of measuring mechanical properties of the meniscus scaffold before an external force is applied to thereto according to the alternate ratio.
FIG. 7 is a drawing showing the evaluation and comparison of stress distribution and deformation according to an alternate ratio during compression of a meniscus scaffold.
FIG. 8 is a graph showing the evaluation and comparison of the peak stress distribution values and deformation values according to an alternate ratio during compression of a meniscus scaffold.

### MODE FOR CARRYING OUT THE INVENTION

Hereinafter, embodiments of the present invention will be described in detail so that a person having ordinary knowledge in the technical field to which the present invention belongs can easily carry out the same with reference to the accompanying drawings.

Embodiments of the present invention to be described below are provided in order to more clearly describe the present invention to a person having ordinary knowledge in the art, and a scope of the present invention is not limited by the following embodiments, and the following embodiments may be modified into various different forms.

Terms used in the present specification are used in order to describe specific embodiments, and are not for limiting the present invention. A term in a singular form used in the present specification may comprise a plural form unless the context clearly indicates otherwise. In addition, terms "comprise" and/or "comprising" used in the present specification specify presence of mentioned shapes, steps, numbers, operations, members, elements, and/or groups thereof, and do not exclude presence or addition of one or more other shapes, steps, numbers, operations, members, elements, and/or groups thereof. In addition, a term "connection" used in the present specification is a concept comprising not only meaning that certain members are directly connected, but also meaning that another member is further interposed between the members and thus indirectly connected.

In addition, when a member is said to be positioned "on" another member in the present specification, this comprises not only a case in which a member is in contact with another member, but also a case in which still another member is present between the two members. The term "and/or" used in the present specification comprises any one of the listed items and all combinations of one or more thereof. In addition, terms of degree such as "about" and "substantially" used in the present specification are used in a sense of the corresponding numerical value or degree or a meaning close thereto in consideration of inherent manufacturing and material tolerances, and are used to prevent an infringer from unfairly exploiting disclosed contents in which exact or absolute numerical values provided to help understanding of the present application are mentioned.

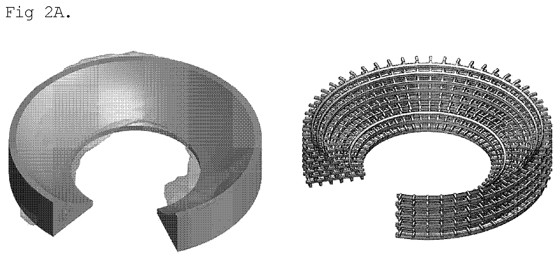

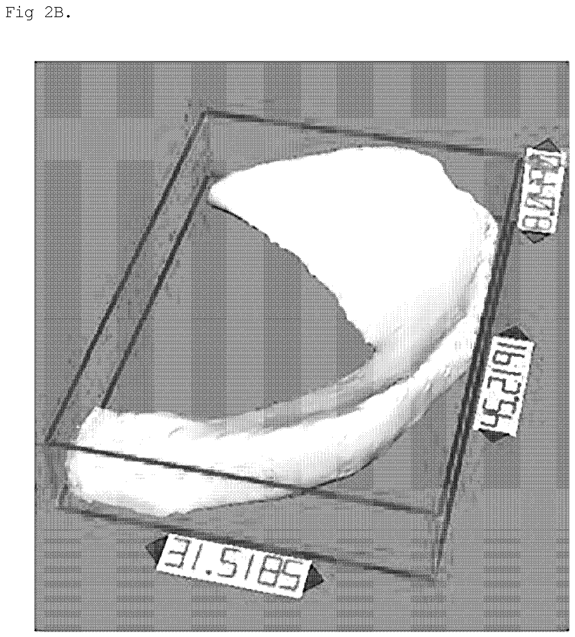

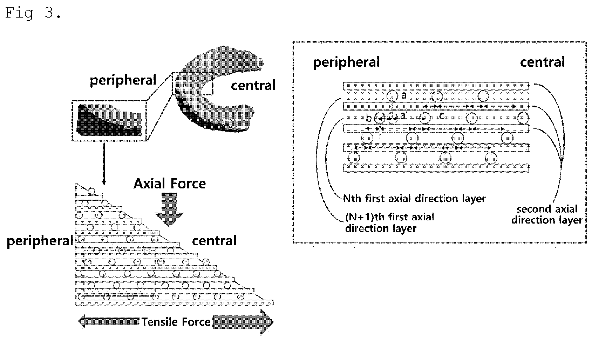

FIG. 2A is a drawing showing a C-shaped porous three-dimensional meniscus scaffold according to one embodiment of the present invention, which mimics a human meniscus, and FIG. 2B is a drawing showing dimensions of the meniscus scaffold shown in FIG. 2A, wherein dimensions of the meniscus scaffold manufactured as one example are 45.2 mm × 31.5 mm × 8.0 mm. FIG. 3 is a drawing showing a cross-sectional view of a meniscus scaffold according to one embodiment of the present invention, and schematically showing an arrangement relationship among an arbitrary first axial direction fiber (a) of an (N+1)th first axial direction layer and a first axial direction fiber (b) and a first axial direction fiber (c) of an Nth first axial direction layer.

Referring to the drawings, a porous three-dimensional meniscus scaffold according to one aspect of the present invention comprises a plurality of first axial direction layers including one or more first axial direction fibers spaced apart from each other at predetermined intervals along a first axial direction; and a plurality of second axial direction layers including one or more second axial direction fibers spaced apart from each other at predetermined intervals along a second axial direction, wherein the first axial direction layers and the second axial direction layers are stacked while crossing each other, wherein, in an (N+1)th first axial direction layer (where N is a natural number of 1 or more, the same applies hereinafter), an arbitrary first axial direction fiber (a) is present, and in an Nth first axial direction layer, a first axial direction fiber (b) first adjacent to the arbitrary first axial direction fiber (a) and a first axial direction fiber (c) second adjacent to the arbitrary first axial direction fiber (a) are presentassuming that the first axial direction fiber (a) is vertically moved to the Nth first axial direction layer as (a'), a ratio of a spacing between the (a') and the (b) to a spacing between the (a') and the (c) is in a range of 1:9 to 3:7.

As such, when the ratio of the spacing between the (a') and the (b) to the spacing between the (a') and the (c) satisfies a range of 1:9 to 3:7, when an external force is applied, stress is not concentrated on any one region and is evenly distributed as compared with a case of departing from the above numerical range, and structurally also shows the least deformation.

At this time, the ratio of the spacing between the (a') and the (b) to the spacing between the (a') and the (c) may specifically be from 1.5:8.5 to 2.5:7.5, and may more specifically be 2:8.

Here, the arbitrary first axial direction fiber and the second axial direction fiber may be stacked orthogonally to each other. At this time, an angle formed by the first axial direction fiber and the second axial direction fiber may be 60° to 120°, 70° to 110°, or 80° to 100°, and may more specifically be about 90°.

In addition, the (b), the (a'), and the (c) may be positioned in order from a peripheral side of the porous three-dimensional meniscus scaffold toward a central side.

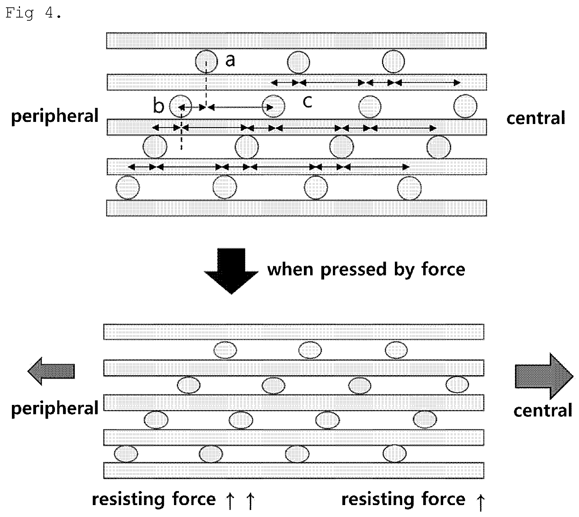

Referring to FIGS. 3 and 4, when an axial force which is an external force is applied to the meniscus scaffold, a tensile force acting on the meniscus scaffold acts more strongly in a central direction, and acts relatively weakly in a peripheral direction. In addition, with respect to a force resisting the external force, the resisting force becomes stronger toward the peripheral direction and weaker toward the central direction. As a result, the applied external force is evenly distributed, a hoop tension is well maintained, and the meniscus can be stably maintained.

Meanwhile, the first axial direction fibers and the second axial direction fibers may independently be a biodegradable polymer, and specifically may comprise at least one polymer selected from the group consisting of polycaprolactone (PCL), Polyglycolic acid (PGA), Polylactic acid (PLA), Poly(lactic-co-glycolic) acid (PLGA), polyurethane (PU), and polydioxanone, and more specifically may be polycaprolactone (PCL), but is not limited thereto.

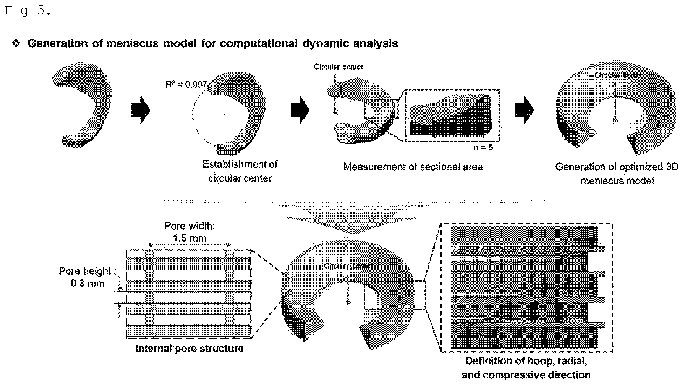

FIG. 5 is a drawing showing a circular center line of an STL 3D model of a meniscus scaffold according to one embodiment of the present invention through image processing, and showing a pore structure formed inside.

Referring to FIG. 5, as a result of calculating a correlation coefficient between a circular center line of the STL 3D model of the meniscus scaffold and the circular function, R2 was 0.997, which is close to 1. Based on this result, it can be confirmed that optimization is possible for a shape obtained by rotating a section of the meniscus scaffold, and optimized 3D modeling can be performed through sections (n=6) of the STL file. Such an optimized model has an advantage in that a section based on the circular center line can be clearly defined, and the same structure can be applied in the entire range.

At this time, a width of pores present inside the porous three-dimensional meniscus scaffold may be 1 mm to 2 mm, 1.25 mm to 1.75 mm, or about 1.5 mm, and a height of the pores may be 0.1 mm to 0.5 mm, 0.2 mm to 0.4 mm, or about 0.3 mm. In addition, a volume fraction of the porous three-dimensional meniscus scaffold may be 15% to 30%, 15% to 25%, or about 20%, and a porosity of the meniscus scaffold may be 70% to 85%, 75% to 85%, or about 80%.

When these numerical ranges are satisfied, an external force applied to the meniscus scaffold can be more stably and evenly distributed, and as a result, a biodegradable medical device comprising such a meniscus scaffold becomes structurally more stable.

The porous three-dimensional meniscus scaffold according to the present invention may be manufactured through a 3D printer.

A line width at the time of output of a polycaprolactone (PCL) polymer through a 3D printer can be controlled through an extrusion pressure. At this time, the line width may correspond to a width of the first axial direction fiber and a width of the second axial direction fiber in the future. When the line width is 270 µm to 330 µm, 280 µm to 320 µm, 290 µm to 310 µm, or about 300 µm, it can be confirmed that a deviation of the extrusion pressure is the lowest.

Therefore, the first axial direction fiber and the second axial direction fiber comprised in the meniscus scaffold according to the present invention may independently have a width of 270 µm to 330 µm, 280 µm to 320 µm, 290 µm to 310 µm, or about 300 µm.

In order to analyze based on Hooke's law and a governing equation, physical properties of PCL can be defined through density of polycaprolactone (PCL) and a strain-stress graph, and it can be confirmed that density of PCL is 1,096 kg m-3, Young's modulus is 320.8 MPa, and plastic properties show a multi-linear result.

Meanwhile, the meniscus scaffold support according to the present invention is characterized in that, when an arbitrary first axial direction fiber of an (N+1)th first axial direction layer is vertically moved to an Nth first axial direction layer, it does not overlap any first axial direction fiber of the Nth first axial direction layer, and such a structure can be defined as an alternate structure. On the contrary, when an arbitraryfirst axial direction fiber of an (N+1)th first axial direction layer is vertically moved to an Nth first axial direction layer and overlaps a first axial direction fiber of the Nth first axial direction layer, such a structure can be defined as a non-alternate structure.

For the cases of the above two groups, compressive stress, radial stress, and hoop stress of each group according to a size of pores inside can be analyzed, and as a result, it can be confirmed that, as the size of the pores increases, both of the above two groups show anisotropy, and it can be confirmed that anisotropy more similar to a living body appears in an alternate structure than in a non-alternate structure.

More specifically, in an alternate structure, since compressive stress can be reduced, it is possible to prevent degeneration of cartilage from additionally occurring after meniscus transplantation.

Meanwhile, the porous three-dimensional meniscus scaffold according to the present invention may further comprise a circumferential fiber formed to surround an outer surface thereof.

By introducing the circumferential fiber, it is possible to make the structure similar to a structure of collagen fibers of a normal meniscus, it is possible to reinforce tensile hoop stress, and thereby, it is possible to form a more stable structure.

That is, when satisfying an alternate structure and comprising all circumferential fibers as in the present invention, a result in which compressive stress decreases and tensile modulus increases is shown, and a more condensed and rigid structure can be exhibited.

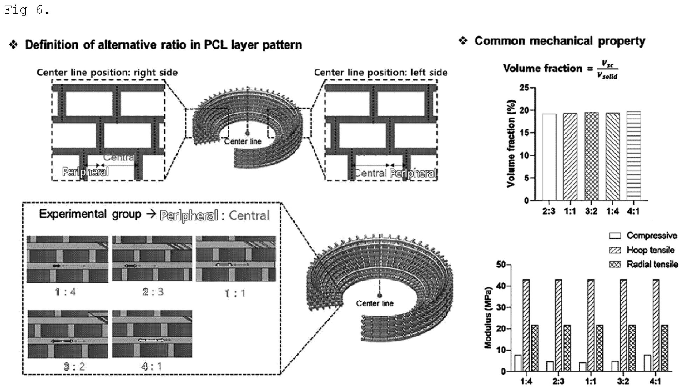

FIG. 6 is a drawing showing that an alternate ratio for having structures of various pores is defined in order to evaluate stress concentration and stability according to a structure of pores formed inside a meniscus scaffold, and showing a result of measuring mechanical properties of the meniscus scaffold before an external force is applied to thereto according to the alternate ratio.

Referring to FIG. 6, since a load of a human body is distributed by a meniscus, it can be expected that there will be an optimal ratio capable of improving stress distribution and stability according to an alternate ratio, and a total of five groups of 1:4, 2:3, 1:1, 3:2, and 4:1 can be defined as ratios according thereto.

Here, the alternate ratio can be defined as a ratio of the spacing between the (a') and the (b) to the spacing between the (a') and the (c) defined in the above-described present invention, and it can be confirmed that all of the above five groups are adjusted to be close to 19.3% in volume fraction (porosity is close to 80.7%), and it can be confirmed that all of the above five groups are maintained without a large difference in anisotropy regardless of the alternate ratio.

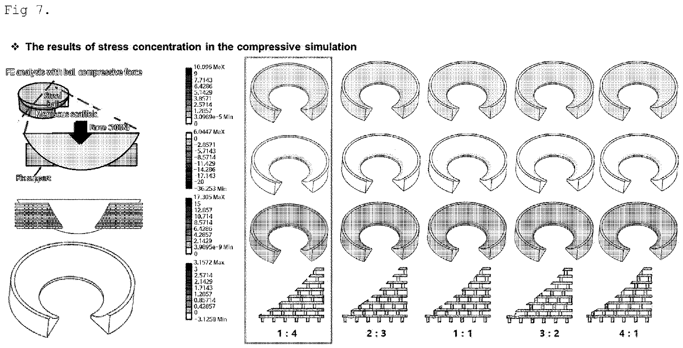

Meanwhile, FIG. 7 is a drawing showing the evaluation and comparison of stress distribution and deformation according to an alternate ratio during compression of a meniscus scaffold.

Here, the applied external force was simulated by being defined as 100 N, not a human body load × 2 (1,150 N), because there are no other knee tissues, that is, femur cartilage, tibial cartilage, tibia, and the like.

As a result of simulation, it can be confirmed that a 1:4 group has the least stress concentration region in all of maximum shear stress, compression stress, equivalent stress (Von-Mises stress), and hoop stress, and further shows the least deformation.

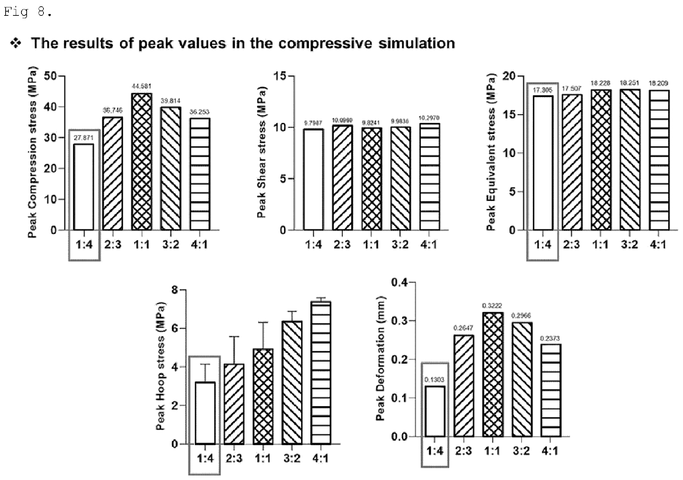

FIG. 8 is a graph showing the evaluation and comparison of the peak stress distribution values and deformation values according to an alternate ratio during compression of a meniscus scaffold.

Referring to FIG. 8, peak stress and deformation of five groups having different alternate ratios were measured in order to predict fracture according to a load, and showed mostly low values in the 1:4 group.

More specifically, in the 1:4 group, peak compression stress was measured as 27.871 MPa, peak shear stress was measured as 9.7987 MPa, peak equivalent stress was measured as 17.305 MPa, peak hoop stress was measured as 3 MPa, and peak deformation was measured as 0.1303 mm, and this result shows a considerably low value as compared with the remaining other groups. By this, it can be confirmed that, when the alternate ratio is about 1:4, the most stable structure is obtained.

Meanwhile, a biodegradable medical device according to another aspect of the present invention is characterized by comprising the above-described porous three-dimensional meniscus scaffold of the present invention.

The medical device may partially contact a living body surface or be implantable into a living body, and may be used to induce regeneration of tissue or cells around the contacted or implanted portion. More specifically, the biodegradable medical device may be a structure for tissue transplantation, a biomimetic tissue, and the like, but is not limited thereto.

In the present specification, preferred embodiments of the present invention have been disclosed, and although specific terms have been used, these are used only in a general sense for easily explaining technical contents of the present invention and helping understanding of the invention, and are not intended to limit a scope of the present invention. It is obvious to a person having ordinary knowledge in the technical field to which the present invention belongs that, in addition to the embodiments disclosed herein, other modified examples based on the technical spirit of the present invention can be practiced. For example, a person having ordinary knowledge in the relevant technical field will be able to know that the porous three-dimensional meniscus scaffold according to the embodiments can be variously modified. Accordingly, the scope of the invention should not be limited by the described embodiments, but should be defined by the claims.

## Claims

1. A porous three-dimensional meniscus scaffold, comprising:
a plurality of first axial direction layers including one or more first axial direction fibers spaced apart from each other at predetermined intervals along a first axial direction; and
a plurality of second axial direction layers including one or more second axial direction fibers spaced apart from each other at predetermined intervals along a second axial direction,
wherein the first axial direction layers and the second axial direction layers are stacked while crossing each other,
wherein, in an (N+1)th first axial direction layer (where N is a natural number of 1 or more, the same applies hereinafter), an arbitrary first axial direction fiber (a) is present, and in an Nth first axial direction layer, a first axial direction fiber (b) first adjacent to the arbitrary first axial direction fiber (a) and a first axial direction fiber (c) second adjacent to the arbitrary first axial direction fiber (a) are present,
assuming that the first axial direction fiber (a) is vertically moved to the Nth first axial direction layer as (a'), a ratio of a spacing between the (a') and the (b) to a spacing between the (a') and the (c) is in a range of 1:9 to 3:7.

2. The porous three-dimensional meniscus scaffold of claim 1, wherein
the arbitrary first axial direction fiber and the second axial direction fiber are stacked orthogonally to each other.

3. The porous three-dimensional meniscus scaffold of claim 1, wherein
the (b), the (a'), and the (c) are positioned in order from a peripheral side of the porous three-dimensional meniscus scaffold toward a central side.

4. The porous three-dimensional meniscus scaffold of claim 1, wherein
the first axial direction fibers and the second axial direction fibers independently comprise at least one polymer selected from the group consisting of polycaprolactone (PCL), Polyglycolic acid (PGA), Polylactic acid (PLA), Poly(lactic-co-glycolic) acid (PLGA), polyurethane (PU), and polydioxanone.

5. The porous three-dimensional meniscus scaffold of claim 1, wherein
a width of pores in the porous three-dimensional meniscus scaffold is 1 mm to 2 mm, and a height of the pores is 0.1 mm to 0.5 mm.

6. The porous three-dimensional meniscus scaffold of claim 1, wherein
a porosity of the porous three-dimensional meniscus scaffold is 70% to 85%.

7. The porous three-dimensional meniscus scaffold of claim 1, wherein
the first axial direction fiber and the second axial direction fiber independently have a width of 270 µm to 330 µm.

8. The porous three-dimensional meniscus scaffold of claim 1, wherein
the porous three-dimensional meniscus scaffold further comprises a circumferential fiber formed to surround an outer surface thereof.

9. The porous three-dimensional meniscus scaffold of claim 1, wherein
the porous three-dimensional meniscus scaffold is manufactured through a 3D printer.

10. A biodegradable medical device comprising the porous three-dimensional meniscus scaffold according to any one of claims 1 to 9.
